# EUROPEAN PATENT APPLICATION

(11) **EP 3 431 579 A1**
(43) Date of publication of application: **23.01.2019**
(21) Application number: 17766462.0
(22) Date of filing: 07.03.2017
(51) Int. Cl.: C12N 1/10, A23K 10/30, A23K 20/105, A23K 50/80, C12N 1/00

(54) **FEED COMPOSITION, METHOD FOR MANUFACTURING ZOOPLANKTON, ZOOPLANKTON, AND ZOOPLANKTON GROWTH PROMOTER AND SURVIVAL RATE ENHANCER**

(30) Priority: 16.03.2016 JP 2016052215
(71) Applicant: Kaneka Corporation, Osaka-shi, Osaka 530-8288 (JP)
(72) Inventor: SATO, Shunsuke, Takasago-shi, Hyogo 676-8688 (JP); OKURA, Tetsuo, Takasago-shi, Hyogo 676-8688 (JP)
(74) Representative: Vossius & Partner Patentanwälte Rechtsanwälte mbB
(86) International application number: PCT/JP2017/009034
(87) International publication number: WO 2017/159461

(57) **Abstract**

Disclosed herein is a feed composition that promotes the growth of zooplankton, enhances the survival rate of zooplankton, is safe, and has little adverse effect on the environment. The feed composition for zooplankton contains PHA.

## Description

### Technical Field

The present invention relates to a feed composition, a method for producing zooplankton, zooplankton, a zooplankton growth promoter, and a zooplankton survival rate enhancer.

### Background Art

In recent years, food problems resulting from population growth have become issues. Particularly, a decrease in the populations of fish and shellfish as natural biological resources due to overfishing has become a problem. Therefore, from the viewpoint of the protection of aquatic resources, farming fishery such as aquaculture has become more important. Zooplankton such as rotifers, copepods, and artemias are essential as starter feed for seedling production of various fish and shellfish. Zooplankton are industrially mass-cultured using algae, such as freshwater chlorella, marine chlorella (Nannochloropsis), and Schizochytrium, as feed and/or nutrient supplements for zooplankton. For example, for the purpose of giving highly unsaturated fatty acids, such as eicosapentaenoic acid (EPA) and docosahexaenoic acid (DHA), that are essential fatty acids for young fish, algae containing these fatty acids may be fed to zooplankton. However, it is known that the amount of the algae fed to zooplankton has a great effect on the growth or survival rate of zooplankton, but there are problems in stably and inexpensively preparing living zooplankton (PTL 1 and PTL 2).

### Citation List

### Patent Literature

PTL 1: WO 2015/159700
PTL 2: JP 2004-147620 A

### Summary of Invention

### Technical Problem

It is an object of the present invention to provide a feed composition that promotes the growth of zooplankton, enhances the survival rate of zooplankton, is safe, and has little adverse effect on the environment.

### Solution to Problem

The present inventors have intensively studied a feed composition that can promote the growth of zooplankton and enhance the survival rate of zooplankton. As a result, the present inventors have surprisingly found that the survival rate of zooplankton can particularly significantly be enhanced by feeding zooplankton with a biodegradable polyester, polyhydroxyalkanoate (hereinafter also referred to as PHA) alone or in combination with a known feed component. Further, the present inventors have found that when both PHA and an alga are fed to zooplankton, the zooplankton can achieve a high growth rate while maintaining a high survival rate. These findings have led to the achievement of the object of the present invention.

More specifically, a first aspect of the present invention relates to a feed composition for zooplankton containing PHA.

The feed composition may contain the PHA and an alga.

In the feed composition, the alga may be at least one of algae belonging to the genus Chlorella, the genus Nannochloropsis, and the genus Schizochytrium.

In the feed composition, the PHA may be at least one selected from the group consisting of PHB, PHBH, and PHBV.

A second aspect of the present invention relates to a method for producing zooplankton, including breeding zooplankton using the feed composition.

A third aspect of the present invention relates to zooplankton bred by feeding the feed composition.

A fourth aspect of the present invention relates to a zooplankton growth promoter containing PHA as an active ingredient.

A fifth aspect of the present invention relates to a zooplankton survival rate enhancer containing PHA as an active ingredient.

In the growth promoter or the survival rate enhancer, the PHA may be at least one selected from the group consisting of PHB, PHBH, and PHBV

### Advantageous Effects of Invention

According to the present invention, it is possible to obtain a feed composition that promotes the growth of zooplankton, enhances the survival rate of zooplankton, is safe, and has little adverse effect on the environment.

### Brief Description of Drawings

Fig. 1 is a graph showing the survival rate of Artemia.
Fig. 2 is a graph showing the total dry weight of Artemia.

### Description of Embodiments

Hereinbelow, the present invention will be described in detail.

### [Feed Composition]

The present invention relates to a feed composition for zooplankton containing PHA. The composition may be PHA alone.

PHA is a generic name for aliphatic polyesters that microorganisms accumulate as energy sources in their cells, and various substances have been reported as monomer components thereof. PHA is originally a natural polymer synthesized by microorganisms from various carbon sources in a natural environment, and is present in soils, seas, and rivers. Further, various microorganisms that decompose and utilize PHA have also been found, and therefore PHA is a material that is part of a normal food chain.

The feed composition according to the present invention uses a biodegradable polyester, PHA and therefore poses no safety risk. In addition, the present inventors have surprisingly found that zooplankton can be bred by feeding PHA alone as feed. This is the first case that the present inventors have found that zooplankton can digest and absorb PHA.

The PHA used in the feed composition for zooplankton according to the present invention is not particularly limited.

In the present invention, the PHA is preferably PHA produced by a microorganism.

Further, in the present invention, the PHA is preferably an aliphatic polyester containing a repeating unit represented by a formula (1): [-CHR-CH₂-CO-O-] (wherein R is an alkyl group represented by CₙH₂ₙ₊₁ and n is an integer of 1 or more but 15 or less).

Further, the PHA is preferably PHA obtained by polymerizing a structural component selected from 3-hydroxyalkanoic acids having 4 to 16 carbon atoms or copolymerized PHA containing a structural unit selected from 3-hydroxyalkanoic acids having 4 to 16 carbon atoms. Examples of such PHAs include polyhydroxybutyrate (PHB) composed of 3-hydroxyalkanoic acid having 4 carbon atoms, polyhydroxybutyratehexanoate (PHBH) composed of 3-hydroxyalkanoic acid having 4 carbon atoms and 3-hydroxyalkanoic acid having 6 carbon atoms, polyhydroxybutyratevalerate (PHBV) composed of 3-hydroxyalkanoic acid having 4 carbon atoms and 3-hydroxyalkanoic acid having 5 carbon atoms, and polyhydroxyalkanoate composed of 3-hydroxyalkanoic acid having 4 to 14 carbon atoms. Among them, at least one selected from the group consisting of PHB, PHBH, and PHBV is preferred, and PHBH or PHBV is more preferred.

Examples of the form of the PHA include, but are not limited to, a cell culture containing cells (bacterium) that produce PHA, dried cells of a bacterium that produces PHA, dried PHA obtained by removing cells and cell components, and a suspension of the dried PHA. It is to be noted that the PHA to be used preferably has such a size that zooplankton can eat.

The feed composition preferably contains PHA and an alga. When the feed composition contains an alga, zooplankton have a high survival rate, and their growth can further be promoted.

Alga is a generic name for organisms that photosynthetically generate oxygen and live in water. The alga is not particularly limited as long as it can be used as feed, but is particularly preferably a planktonic alga, except for large algae, that lives in fresh water or seawater and has a size of 1 to 1,000 µm. Examples of such an alga include: algae belonging to the division Chlorophyta such as Chlorella, Dunaliella, and Parachlorella; algae belonging to the division Heterokontophyta such as Nannochloropsis; algae belonging to the division Cyanophyta such as Spirulina and Arthrospira; algae belonging to the class Labyrinthulea such as Shizochytrium and Aurantichytrium; and algae belonging to the division Euglenophyta such as Euglena. Among these algae, those containing a nutrient enhancement component such as a highly unsaturated fatty acid (e.g., DHA or EPA) are particularly preferred from the viewpoint of promoting the growth of zooplankton and enhancing the survival rate of zooplankton. More specifically, the alga is, for example, at least one of algae belonging to the genus Chlorella, the genus Nannochloropsis, and the genus Schizochytrium.

The alga can be used in the form of a culture medium in which the alga has been cultured, a dried product obtained by drying the alga, a product obtained by grinding the culture medium and the dried product, or a culture suspension of the alga.

The feed composition containing PHA and an alga can be obtained by, for example, mixing the alga and the PHA. When the alga and the PHA are mixed, their forms are not particularly limited. For example, the PHA and the alga may be in the form of a dried product or a suspension.

The feed composition may further contain a feed component other than the PHA and the alga. Such a feed component is not particularly limited, and any known feed component can be used. Examples of the known feed component include highly unsaturated fatty acids (e.g., eicosapentaenoic acid (EPA), docosahexaenoic acid (DHA), and docosapentaenoic acid (DPA)).

By feeding the feed composition according to the present invention to zooplankton, it is possible to quickly grow zooplankton while maintaining a high survival rate. Living zooplankton can absorb highly unsaturated fatty acids contained in algae such as Chlorella, Nannochloropsis, and Schizochytrium. Therefore, when such algae are fed to zooplankton having a high survival rate, the zooplankton has greater value as feed than zooplankton having a low survival rate. As described above, the feed composition according to the present invention makes it possible to efficiently and stably obtain nutritious zooplankton.

The species of zooplankton are not particularly limited as long as the effect of enhancing the survival rate and the growth rate can be obtained by feeding the feed composition according to the present invention to the zooplankton as feed.

Zooplankton is a generic name for microorganisms that eat organic matter such as phytoplankton as food without performing photosynthesis and float in water, but particularly refers to microorganisms, such as rotifers, artemias, copepods, and water fleas, that are generally used as live feed or feed for fish and shellfish in seedling production or aquaculture. Rotifers are organisms that belong to the phylum Rotifera and include Brachionus plicatilis and S-type rotifers (B. rotundiformis). Artemias are organisms that belong to the phylum Arthropod and the subphylum Crustacea and include Artemia salina. Copepods and water fleas are organisms that belong to the phylum Arthropod.

It is to be noted that the feed composition according to the present invention may be fed as feed not only to zooplankton but also to aquatic organisms such as fish, seashells, and shellfish. Examples of the fish include, but are not limited to, small fish such as killifish (Oryzias javanicus) and sardines, red sea breams, black sea breams, flatfish, tiger pufferfish, octopuses, adult yellowtails, codfish, and salmons. Examples of the seashells include, but are not limited to, Japanese oysters and mussels. Examples of the shellfish include, but are not limited to, shrimps and crabs.

### [Production of zooplankton]

A method for producing zooplankton according to the present invention includes breeding zooplankton using the feed composition described above. Specific examples of the use of the feed composition include feeding zooplankton with PHA alone as the feed composition; feeding zooplankton with the feed composition containing an alga and PHA, and feeding zooplankton with PHA and an alga, which are separately prepared, at the same time or at different times. The PHA and the alga may be in the form of a dried product or a suspension, and their forms are not particularly limited.

The optimum mixing ratio between the alga and the PHA or the optimum use ratio between the alga and the PHA (use ratio refers to the ratio between the amount of the alga and the amount of the PHA at the time when the alga and the PHA are fed at the same time or different times) varies depending on the species of an organism to which the PHA and the alga are to be fed and may be appropriately set. For example, the amount of the PHA is preferably adjusted to 0.1 to 50 wt%, more preferably 1 to 50 wt%, even more preferably 5 to 50 wt% with respect to the total weight of the alga and the PHA.

The timing for feeding the feed composition according to the present invention to an aquatic organism is not particularly limited. The feed composition according to the present invention may be fed during a period from just after hatching until larvae grow to desired individuals or may be fed only for a certain period of time. The specific feeding period varies depending on the type of target aquatic organism, but is, for example, 2 days or more, preferably 3 days or more, more preferably 5 days or more. The upper limit is not particularly limited either, and the feed composition according to the present invention may be fed for several weeks or several months. More specifically, the effects of the present invention can be obtained even by feeding the feed composition according to the present invention for, for example, about 3 to 7 days.

Zooplankton bred by feeding the feed composition according to the present invention containing PHA have a higher survival rate than those bred by feeding an alga that is a commercially-available feed/nutrient supplement for zooplankton. Therefore, the feed composition according to the present invention containing PHA can be used also as a survival rate enhancer for aquatic organisms such as zooplankton.

PHA contains no highly unsaturated fatty acids essential for the growth of young fish. Therefore, PHA may be fed to zooplankton in combination with an alga for the purpose of nutrient enhancement, in which case a higher growth rate as well as a higher survival rate can be achieved as compared with a case where PHA or an alga is fed alone. That is, the feed composition according to the present invention containing PHA, preferably the feed composition containing PHA and an alga can be used also as a growth promoter for aquatic organisms such as zooplankton.

A technique for efficiently and stably producing living zooplankton is important for aquaculture of fish, shellfish, seashells, and the like using the zooplankton as feed. Further, PHA used in the present invention is biodegradable, and particularly, exhibits higher biodegradability even in water than other biodegradable plastics. Therefore, the feed composition according to the present invention is excellent also in that it is safe and has little adverse effect on the environment.

### [Zooplankton]

The aquatic organisms such as zooplankton obtained by feeding the feed composition according to the present invention have a high growth rate and a high survival rate, and therefore can be stably and inexpensively supplied as feed for aquaculture of fish and shellfish. Further, the aquatic organisms can have a high nutritional value, and therefore also has great value as feed.

### Examples

Hereinbelow, the present invention will be more specifically described with reference to examples. However, the present invention is not limited thereto.

### (Example 1) Test in which PHBH was fed alone to Artemia salina

In this test, hatched Artemia salina (48 hours old or more) after mouth opening was used. A 1-liter beaker was used as a test container, and the density of Artemia salina was set to 50,000 individuals/liter. Filtered natural seawater (salt concentration: 3.2%) was used as a test water, and the temperature of the seawater was adjusted to 25°C by a plate heater provided under the test container. Further, feed was fed three times in total after 0, 20, and 40 hours from the start of the test. The feed was fed in an amount of 40 mg per 50,000 individuals/L each time. The number of lots for repeated measurement was 3. It is to be noted that prior to the feeding after 20 hours from the start of the test and the feeding after 40 hours from the start of the test, Artemia salina was collected with a plankton net (opening: 125 µm), and all the remaining breeding water was replaced with the same test water as described above.

The feed used in the test was a PHBH powder (manufactured by Kaneka Corporation under the trade name of Kaneka Biopolymer AONILEX (trademark)) whose average particle size (MV) was about 2.95 µm that was small enough for zooplankton to eat.

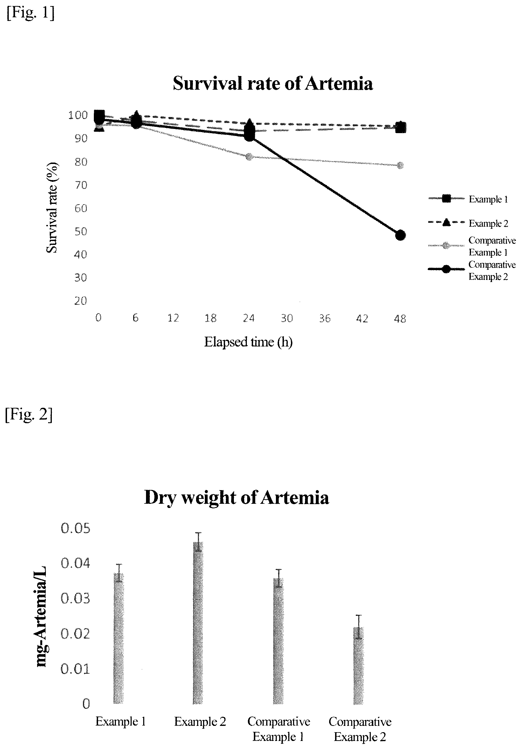

Sampling was perfonned after 0, 6, 24, and 48 hours from the start of the test, and the total number of individuals and the number of surviving individuals per 500 µL were measured with a microscope. The survival rate (%) of Artemia was calculated as the number of surviving individuals/the total number of individuals × 100. The determination as to whether or not the individuals were surviving individuals was made by visually observing the motility of the individuals. Further, after 48 hours from the start of the test (after the test), all the individuals of Artemia salina were collected with a plankton net (opening: 125 µm), washed with water twice, and freeze-dried to obtain dried individuals. The weight of the dried individuals (total dry weight of Artemia) was measured. The survival rate of Artemia is shown in Table 1 and Fig. 1, and the total dry weight of Artemia after test is shown in Fig. 2.

As a result, it was confirmed that Artemia salina grew without any problem by feeding PHBH alone (Table 1 and Fig. 1) and that the total dry weight of Artemia after the completion of the test was comparable to that of Comparative Example 1 (that will be described later) in which Schizochytrium was fed alone. From the result, it was found that Artemia salina bred by feeding PHBH alone had a growth rate equal to or higher than that of Artemia salina bred by feeding an alga (Fig. 2). Further, the survival rate of Artemia was significantly higher than those of test groups (Comparative Examples 1 and 2) in which PHBH was not fed.

### (Example 2) Test in which mixed feed of PHBH and Schizochytrium was fed to Artemia salina

A test was performed in the same manner as in Example 1 except that mixed feed of PHBH (PHBH powder (manufactured by Kaneka Corporation under the trade name of Kaneka Biopolymer AONILEX (trademark)) and Schizochytrium was used as the feed instead of the PHBH powder. The mixing ratio between PHBH and Schizochytrium was 1 : 1 (weight ratio). As the Schizochytrium, Bio-Chromis (manufactured by PACIFIC TRADING CO., LTD.) was used. The survival rate of Artemia is shown in Table 1 and Fig. 1, and the total dry weight of Artemia after test is shown in Fig. 2.

As a result, it was confirmed that the total dry weight of Artemia after the completion of the test was heavier than that of Example 1 in which PHBH was fed alone or that of Comparative Example 1 (that will be described later) in which Schizochytrium was fed alone, that is, the growth rate of Artemia was higher than those of Example 1 and Comparative Example 1 (Fig. 2). Further, the survival rate of Artemia was significantly higher than those of test groups (Comparative Examples 1 and 2) in which PHBH was not fed.

### (Comparative Example 1) Test in which Schizochytrium was fed alone to Artemia salina

A test was performed in the same manner as in Example 1 except that Schizochytrium (product name: Bio-Chromis) was used alone as the feed instead of the PHBH powder. The survival rate of Artemia is shown in Table 1 and Fig. 1, and the total dry weight of Artemia after test is shown in Fig. 2.

As a result, the growth rate (the total dry weight of Artemia after the completion of the test) was comparable to that of Example 1 in which PHBH was fed alone, but the survival rate of Artemia was reduced during breeding, and was therefore as low as about 80% after 48 hours from the start of the test.

### (Comparative Example 2) Test in which feed was not given

A growth test and a survival rate test were performed on Artemia in the same manner as in Example 1 except that the feed was not given at all. The survival rate of Artemia is shown in Table 1 and Fig. 1, and the total dry weight of Artemia after test is shown in Fig. 2.

As a result, the survival rate was started to reduce after 24 hours from the start of the test, and about five-tenths of individuals of Artemia were dead after 48 hours. The total dry weight of Artemia after the completion of the test was significantly lower (the growth rate was lower) than those of other test groups. It is considered that the total dry weight of Artemia after the completion of the test is close to the total dry weight of Artemia at the start of the test because feed was not given during the test period in the test group of this comparative example.

**[Table 1]**

| Elapsed time (h) | Lot. | | Example 1 (PHBH-feeding group) | | Example 2 (PHBH/Schizochytrium-feeding group) | | Comparative Example 1 (Schizochytrium-feeding group) | | Comparative Example 2 (Non-feeding group) | |
|---|---|---|---|---|---|---|---|---|---|---|
| | | | Number of individuals | Survival Rate (%) | Number of individuals | Survival Rate (%) | Number of individuals | Survival Rate (%) | Number of individuals | Survival Rate (%) |
| 0 | 1 | Number of surviving individuals | 10 | 100.0 | 22 | 91.7 | 28 | 96.6 | 19 | 95.0 |
| | | Total number of individuals | 10 | | 24 | | 29 | | 20 | |
| | 2 | Number of surviving individuals | 24 | 100.0 | 17 | 100.0 | 19 | 95.0 | 23 | 100.0 |
| | | Total number of individuals | 24 | | 17 | | 20 | | 23 | |
| | 3 | Number of surviving individuals | 21 | 100.0 | 16 | 94.1 | 24 | 96.0 | 28 | 100.0 |
| | | Total number of individuals | 21 | | 17 | | 25 | | 28 | |
| | Average survival rate (%) | | 100.0 | | 95.3 | | 95.9 | | 98.3 | |
| 6 | 1 | Number of surviving individuals | 14 | 100.0 | 14 | 100.0 | 19 | 95.0 | 26 | 100.0 |
| | | Total number of individuals | 14 | | 14 | | 20 | | 26 | |
| | 2 | Number of surviving individuals | 14 | 93.3 | 21 | 100.0 | 22 | 91.7 | 20 | 100.0 |
| | | Total number of individuals | 15 | | 21 | | 24 | | 20 | |
| | 3 | Number of surviving individuals | 23 | 100.0 | 20 | 100.0 | 22 | 100.0 | 18 | 90.0 |
| | | Total number of individuals | 23 | | 20 | | 22 | | 20 | |
| | Average survival rate (%) | | 97.8 | | 100.0 | | 95.6 | | 96.7 | |
| 24 | 1 | Number of surviving individuals | 21 | 95.5 | 13 | 100.0 | 13 | 76.5 | 17 | 73.9 |
| | | Total number of individuals | 22 | | 13 | | 17 | | 23 | |
| | 2 | Number of surviving individuals | 12 | 100.0 | 18 | 90.0 | 21 | 91.3 | 19 | 100.0 |
| | | Total number of individuals | 12 | | 20 | | 23 | | 19 | |
| | 3 | Number of surviving individuals | 17 | 85.0 | 20 | 100.0 | 20 | 80.0 | 18 | 100.0 |
| | | Total number of individuals | 20 | | 20 | | 25 | | 18 | |
| | Average survival rate (%) | | 93.5 | | 96.7 | | 82.6 | | 91.3 | |
| 48 | 1 | Number of surviving individuals | 17 | 89.5 | 15 | 100.0 | 14 | 82.4 | 7 | 43.8 |
| | | Total number of individuals | 19 | | 15 | | 17 | | 16 | |
| | 2 | Number of surviving individuals | 17 | 100.0 | 13 | 92.9 | 12 | 66.7 | 11 | 57.9 |
| | | Total number of individuals | 17 | | 14 | | 18 | | 19 | |
| | 3 | Number of surviving individuals | 25 | 96.2 | 18 | 94.7 | 15 | 88.2 | 10 | 45.5 |
| | | Total number of individuals | 26 | | 19 | | 17 | | 22 | |
| | Average survival rate (%) | | 95.2 | | 95.9 | | 79.1 | | 49.0 | |

## Claims

1. A feed composition for zooplankton, comprising PHA.

2. The feed composition according to claim 1, comprising the PHA and an alga.

3. The feed composition according to claim 2, wherein the alga is at least one of algae that belongs to the genus Chlorella, the genus Nannochloropsis, and the genus Schizochytrium.

4. The feed composition according to any one of claims 1 to 3, wherein the PHA is at least one selected from the group consisting of PHB, PHBH, and PHBV.

5. A method for producing zooplankton, comprising breeding zooplankton using the feed composition according to any one of claims 1 to 4.

6. Zooplankton bred by feeding the feed composition according to any one of claims 1 to 4.

7. An agent for promoting growth of zooplankton, comprising PHA as an active ingredient.

8. An agent for enhancing a survival rate of zooplankton, comprising PHA as an active ingredient.

9. The agent according to claim 7 or 8, wherein the PHA is at least one selected from the group consisting of PHB, PHBH, and PHBV
